# EUROPEAN PATENT APPLICATION

(11) **EP 4 365 669 A1**
(43) Date of publication of application: **08.05.2024**
(21) Application number: 22833005.6
(22) Date of filing: 24.06.2022
(51) Int. Cl.: G02C 13/00

(54) **SOFT CONTACT LENS SOLUTION**

(30) Priority: 29.06.2021 JP 2021107493
(71) Applicant: NOF Corporation, Shibuya-ku Tokyo 150-6019 (JP)
(72) Inventor: KAWASAKI Hiroko, Kawasaki-shi, Kanagawa 210-0865 (JP); IWAKIRI Norio, Kawasaki-shi, Kanagawa 210-0865 (JP)
(74) Representative: Mewburn Ellis LLP
(86) International application number: PCT/JP2022/025211
(87) International publication number: WO 2023/276858

(57) **Abstract**

Provided is a soft contact lens solution that imparts surface hydrophilicity, lubricity, and a lipid adhesion-suppressing ability to a soft contact lens. It has been found that a solution containing two specific kinds of copolymers and a buffering agent can achieve the above-mentioned object. Thus, the soft contact lens solution of the present disclosure has been completed.

## Description

### Technical Field

The present disclosure relates to a soft contact lens solution that imparts surface hydrophilicity, lubricity, and a lipid adhesion-suppressing ability to a soft contact lens.

The present application claims priority from Japanese Patent Application No. 2021-107493, which is incorporated herein by reference.

### Background Art

The number of contact lens wearers is rapidly increasing because of the ease and convenience of a contact lens, and the contact lens has become a medical device that is widely and generally used at present. However, because of discomfort associated with wearing of contact lenses, there is a large number of cases of discontinuation of wearing, and it cannot be said that sufficient research has been conducted on improvement of wearing comfort.

It has been known that wettability and an antifouling property are closely related to the above-mentioned discomfort, and there is a demand for a method of improving the surface hydrophilicity of a contact lens, a method of imparting an antifouling property, and the like. In addition, in recent years, a friction coefficient of a surface of a contact lens has also been regarded important, and a contact lens having a high friction coefficient may cause not only discomfort during wearing but also conjunctivitis and the like (see: Non Patent Literature 1 and Non Patent Literature 2).

In order to solve the above-mentioned problems, a method involving subjecting a contact lens to surface treatment, and the like have been developed. For example, there are disclosed a method involving subjecting a hydrophilic monomer to graft polymerization on a surface of a plasma-treated contact lens, to thereby impart surface hydrophilicity and an antifouling property to the surface of the contact lens, and the like (Patent Literature 1). In addition, there is disclosed a method of producing a contact lens having high hydrophilicity and high lubricity by coating a surface of a contact lens with a polymer (Patent Literature 2).

However, these surface treatments require complicated steps and are not desired for mass production.

### Citation List

### Patent Literature

[PTL 1] JP 2018-022174 A
[PTL 2] JP 06-122779 A

### Non Patent Literature

[NPL 1] Roba, M. et al., Friction Measurements on Contact Lenses In Their Operating Environment, Tribol. Lett., 2011, 44(3), 387-397.
[NPL 2] Norihiko Yokoi, Mechanisms of Eye Dryness Due to Contact Lens Wear, Journal of Japan Contact Lens Society, 2009, 51(3), S33-S35.

### Summary of Invention

### Technical Problem

An object of the present disclosure is to provide a soft contact lens solution that imparts surface hydrophilicity, lubricity, and a lipid adhesion-suppressing ability to a soft contact lens.

### Solution to Problem

The inventors have made extensive investigations in order to achieve the above-mentioned object. As a result, the inventors have found that a solution containing two specific kinds of copolymers and a buffering agent can achieve the above-mentioned object. Thus, the inventors have completed a soft contact lens solution of the present disclosure.
1. A soft contact lens solution, including: 0.001 w/v% to 1.0 w/v% of a copolymer (P) containing constituent units represented by the following formulae (1a) to (1c), having a ratio "na:nb:nc" of numbers of the constituent units of 100:10 to 400:2 to 50, and having a weight-average molecular weight of from 5,000 to 2,000,000; 0.002 w/v% to 2.0 w/v% of a copolymer (Q) being a polyoxyethylene-polyoxypropylene block copolymer, having a number of moles added of ethylene oxide (EO) of from 120 to 200 and a number of moles added of propylene oxide (PO) of from 10 to 70, and having a content of the EO of from 60 wt% to 90 wt%; and a buffering agent: 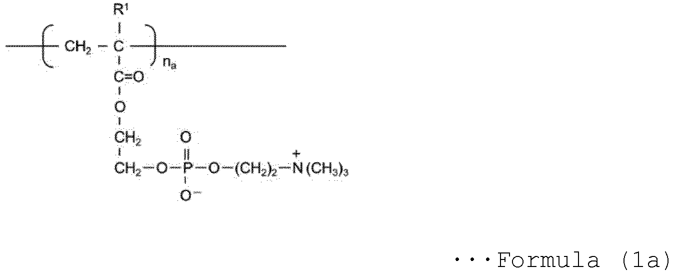 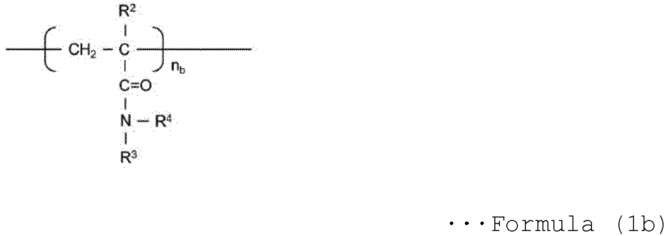 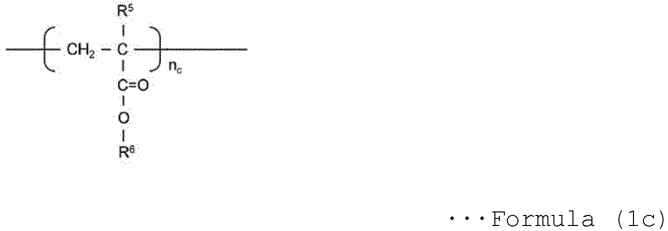 where R¹, R², and R⁵ each independently represent a hydrogen atom or a methyl group, R³ and R⁴ each independently represent a hydrogen atom, a methyl group, or an ethyl group, or are bonded to each other to represent a morpholino group, and R⁶ represents a monovalent hydrocarbon group having 12 to 24 carbon atoms.
2. The soft contact lens solution according to the above-mentioned item 1, wherein the buffering agent is a phosphate buffer.
3. The soft contact lens solution according to the above-mentioned item 1 or 2, wherein the constituent unit represented by the formula (1a) is a constituent unit based on 2-(methacryloyloxy)ethyl 2-(trimethylammonio)ethyl phosphate, the constituent unit represented by the formula (1b) is a constituent unit based on N,N-dimethylacrylamide, and the constituent unit represented by the formula (1c) is a constituent unit based on stearyl methacrylate.
4. The soft contact lens solution according to the above-mentioned item 1 or 2, wherein the constituent unit represented by the formula (1a) is a constituent unit based on 2-(methacryloyloxy)ethyl 2-(trimethylammonio)ethyl phosphate, the constituent unit represented by the formula (1b) is a constituent unit based on N,N-dimethylacrylamide, and the constituent unit represented by the formula (1c) is a constituent unit based on lauryl methacrylate.
5. A surface treatment method for a soft contact lens, including a step of bringing a soft contact lens solution into contact with a surface of a soft contact lens, the soft contact lens solution including: 0.001 w/v% to 1.0 w/v% of a copolymer (P) containing constituent units represented by the following formulae (1a) to (1c), having a ratio "na:nb:nc" of numbers of the constituent units of 100:10 to 400:2 to 50, and having a weight-average molecular weight of from 5,000 to 2,000,000; 0.002 w/v% to 2.0 w/v% of a copolymer (Q) being a polyoxyethylene-polyoxypropylene block copolymer, having a number of moles added of ethylene oxide (EO) of from 120 to 200 and a number of moles added of propylene oxide (PO) of from 10 to 70, and having a content of the EO of from 60 wt% to 90 wt%; and a buffering agent: 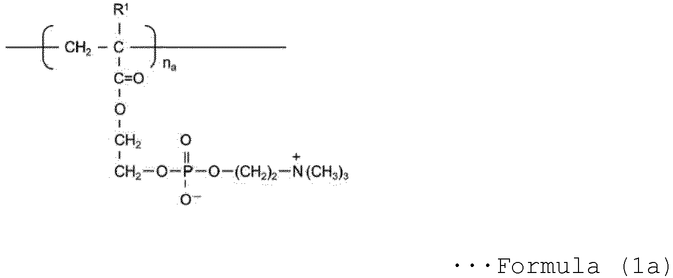 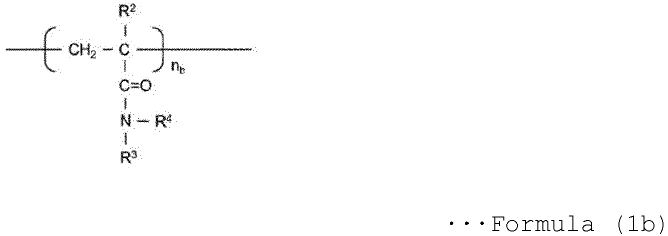 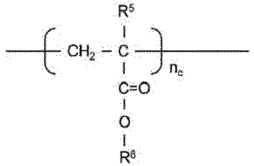 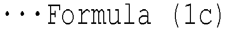 where R¹, R², and R⁵ each independently represent a hydrogen atom or a methyl group, R³ and R⁴ each independently represent a hydrogen atom, a methyl group, or an ethyl group, or are bonded to each other to represent a morpholino group, and R⁶ represents a monovalent hydrocarbon group having 12 to 24 carbon atoms.
6. A use of: a copolymer (P) containing constituent units represented by the following formulae (1a) to (1c), having a ratio "na:nb:nc" of numbers of the constituent units of 100:10 to 400:2 to 50, and having a weight-average molecular weight of from 5,000 to 2,000,000; a copolymer (Q) being a polyoxyethylene-polyoxypropylene block copolymer, having a number of moles added of ethylene oxide (EO) of from 120 to 200 and a number of moles added of propylene oxide (PO) of from 10 to 70, and having a content of the EO of from 60 wt% to 90 wt%; and a buffering agent, for production of a soft lens solution: 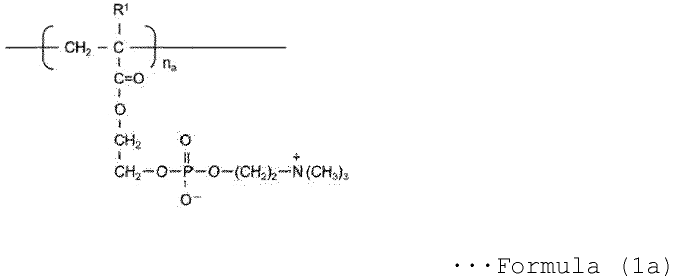 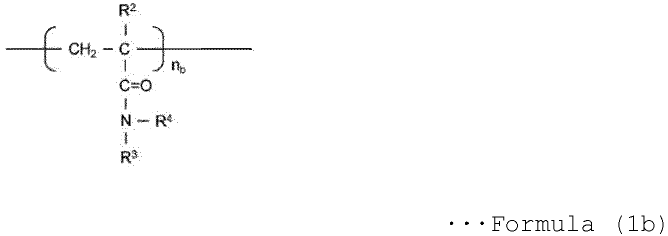 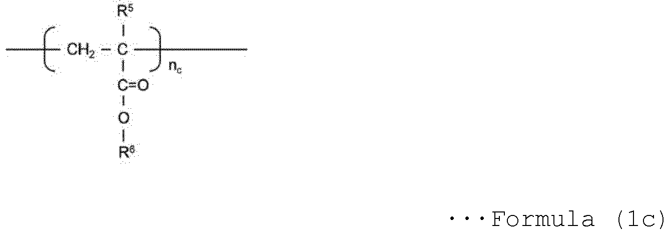 where R¹, R², and R⁵ each independently represent a hydrogen atom or a methyl group, R³ and R⁴ each independently represent a hydrogen atom, a methyl group, or an ethyl group, or are bonded to each other to represent a morpholino group, and R⁶ represents a monovalent hydrocarbon group having 12 to 24 carbon atoms.

### Advantageous Effects of Invention

The soft contact lens solution of the present disclosure can impart surface hydrophilicity, lubricity, and a lipid adhesion-suppressing ability to a soft contact lens.

### Description of Embodiments

A soft contact lens solution of the present disclosure includes: 0.001 w/v% to 1.0 w/v% of a copolymer (P) containing constituent units represented by the following general formulae (1a) to (1c), having a ratio "na:nb:nc" of numbers of the constituent units of 100:10 to 400:2 to 50, and having a weight-average molecular weight of from 5,000 to 2,000,000; 0.002 w/v% to 2.0 w/v% of a copolymer (Q) being a polyoxyethylene-polyoxypropylene block copolymer, having a number of moles added of ethylene oxide (EO) of from 120 to 200 and a number of moles added of propylene oxide (PO) of from 10 to 70, and having a content of the EO of from 60 wt% to 90 wt%; and a buffering agent.

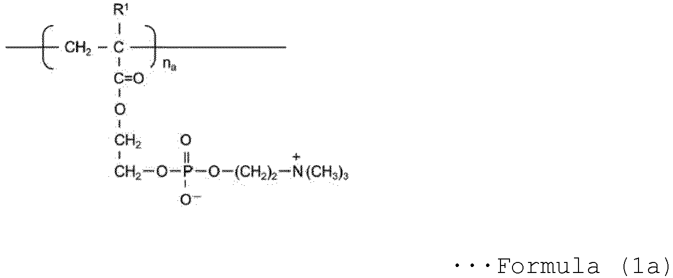

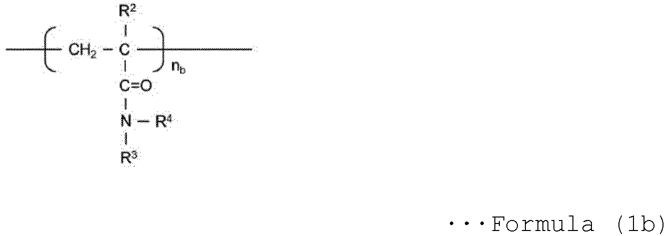

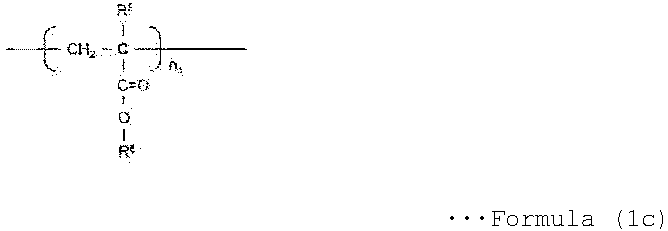

R¹, R², and R⁵ each independently represent a hydrogen atom or a methyl group, R³ and R⁴ each independently represent a hydrogen atom, a methyl group, or an ethyl group, or are bonded to each other to represent a morpholino group, and R⁶ represents a monovalent hydrocarbon group having 12 to 24 carbon atoms.

A configuration of the soft contact lens solution of the present disclosure is described below.

As used herein, the term "(meth)acrylate" means "acrylate or methacrylate, " and the same applies to the other similar terms.

In addition, as used herein, when a preferred numerical range (e.g., range of concentration or weight-average molecular weight) is described in stages, respective lower limit values and upper limit values may be independently combined with each other. For example, in the description "preferably 10 or more, more preferably 20 or more, and preferably 100 or less, more preferably 90 or less," the "preferred lower limit value: 10" and the "more preferred upper limit value: 90" may be combined to be referred as "10 or more and 90 or less." In addition, for example, the description "preferably from 10 to 100, more preferably from 20 to 90" may be similarly referred to as "from 10 to 90."

### <Copolymer (P)>

The copolymer (P) is a copolymer containing constituent units represented by the general formulae (1a) to (1c), having a ratio "na:nb:nc" of numbers of the constituent units of 100:10 to 400:2 to 50, and having a weight-average molecular weight of from 5,000 to 2,000,000.

The constituent unit means a unit of a compound included in a polymer based on each monomer or derived from each monomer.

### [Constituent Unit represented by General Formula (1a)]

The copolymer (P) contains a constituent unit having a phosphorylcholine structure of the following formula (1a) (hereinafter also referred to as "PC constituent unit"). When the copolymer (P) contains a PC constituent unit, the copolymer (P) has hydrophilicity and can impart lubricity and a lipid adhesion-suppressing ability to a contact lens.

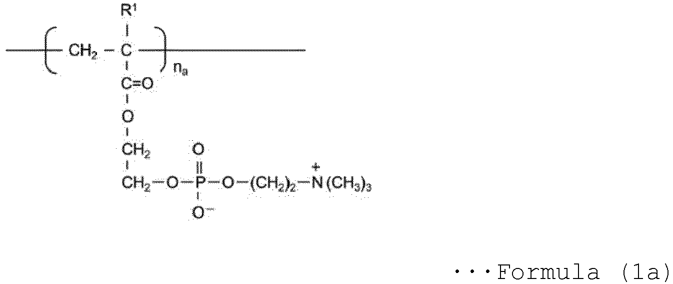

The R¹ represents a hydrogen atom or a methyl group.

The copolymer (P) containing the PC constituent unit may be obtained, for example, by copolymerizing a phosphorylcholine group-containing monomer represented by the following general formula (1a') (hereinafter also referred to as "PC monomer").

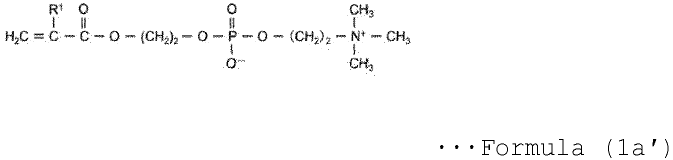

From the viewpoint of ease of availability, the PC monomer is preferably 2-((meth)acryloyloxy)ethyl-2-(trimethylammonio)ethyl phosphate, more preferably 2-(methacryloyloxy)ethyl-2-(trimethylammonio)ethyl phosphate represented by the following formula (1a") (hereinafter also referred to as "2-methacryloyloxyethyl phosphorylcholine").

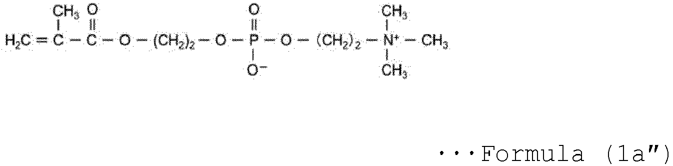

The PC monomer may be produced by a known method. For example, reference may be made to a method described in JP 54-63025. There is given a method involving subjecting a hydroxy group-containing polymerizable monomer and 2-bromoethylphosphoryl dichloride to a reaction in the presence of a tertiary base, and then causing the resultant compound to react with a tertiary amine. In addition, reference may also be made to a method described in JP 58-154591 or the like. There are given a method involving obtaining a cyclic compound by a reaction between a hydroxy group-containing polymerizable monomer and a cyclic phosphorus compound, followed by a ring-opening reaction with a tertiary amine, and the like.

### [Constituent Unit represented by General Formula (1b)]

The copolymer (P) contains a constituent unit represented by the following general formula (1b) (hereinafter also referred to as "amide constituent unit"). When the copolymer (P) contains an amide constituent unit, the adhesiveness of the copolymer (P) to a contact lens can be improved.

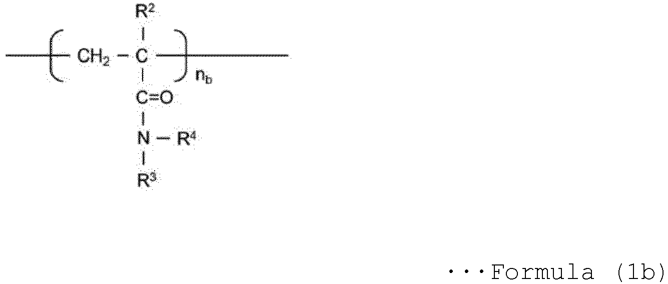

In the formula (1b), R² represents a hydrogen atom or a methyl group, and R³ and R⁴ each independently represent a hydrogen atom, a methyl group, or an ethyl group, or are bonded to each other to represent a morpholino group.

A ratio of the amide constituent unit in the copolymer (P) is as described below. Regarding a ratio n_{b} of the number of the amide constituent units, when a ratio nₐ of the number of the PC constituent units is set to 100, a ratio "n_{b}/nₐ" is from 10 to 400/100, preferably from 30 to 250/100. When the n_{b} is too large, biocompatibility is not sufficient. When the n_{b} is too small, the intended effects are not expected to be imparted to a contact lens.

The amide constituent unit in the copolymer (P) is obtained from (meth)acrylamide or a (meth)acrylamide derivative that is a monomer represented by the following formula (1b') to be used at the time of polymerization of the copolymer (P).

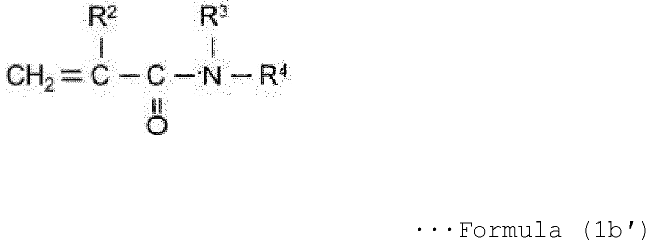

R², R³, and R⁴ in the formula (1b') are the same as R², R³, and R⁴ in the formula (1b), respectively.

Examples of (meth)acrylamide or the (meth)acrylamide derivative represented by the formula (1b') include N,N-dimethyl (meth)acrylamide, N,N-diethyl (meth)acrylamide, and N-acryloyl morpholine. A preferred example of the amide constituent unit may be N,N-dimethyl acrylamide.

### [Constituent Unit represented by General Formula (1c)]

The copolymer (P) contains a constituent unit represented by the following general formula (1c) (hereinafter also referred to as "hydrophobic constituent unit"). The hydrophobic constituent unit in the copolymer (P) is introduced in order to increase adsorptivity to a contact lens and impart the intended effects to the contact lens.

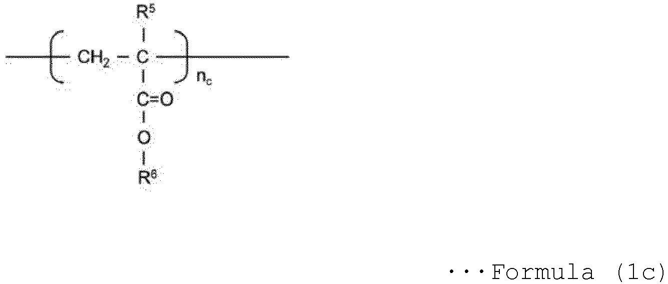

In the formula (1c), R⁵ represents a hydrogen atom or a methyl group, and R⁶ represents a monovalent hydrocarbon group having 12 to 24, 12 to 22, or 12 to 18 carbon atoms.

R⁶ may be linear or branched, but is preferably linear. Specific examples thereof include a lauryl group, a stearyl group, and a behenyl group.

A ratio of the hydrophobic constituent unit in the copolymer (P) is as described below. Regarding a ratio n_{c} of the number of the hydrophobic constituent units, when the ratio nₐ of the number of the PC constituent units is set to 100, a ratio "n_{c}/nₐ" is from 2 to 50/100, preferably from 5 to 25/100. When the n_{c} is too large, the hydrophilicity of the copolymer (P) is decreased to lower the solubility in an aqueous solution, and it may be difficult to produce a contact lens solution. When the n_{c} is too small, a physical cross-linking gel forming ability based on a hydrophobic interaction is decreased to lower the adhesiveness to the surface of a contact lens, and the intended effects may not be continuously imparted to a contact lens.

The hydrophobic constituent unit in the copolymer (P) is obtained from a hydrophobic monomer represented by the following formula (1c') to be used at the time of polymerization of the copolymer (P).

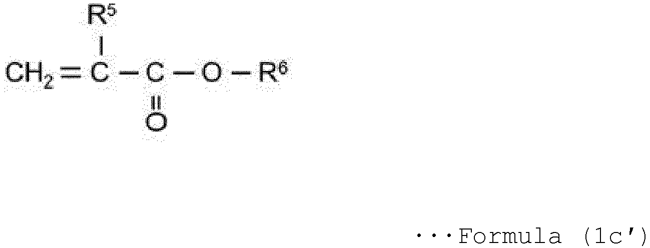

R⁵ and R⁶ in the formula (1c') are the same as R⁵ and R⁶ in the formula (1c), respectively.

Examples of the hydrophobic monomer represented by the formula (1c') include linear alkyl (meth)acrylates, such as lauryl (meth)acrylate, stearyl (meth)acrylate, and behenyl (meth)acrylate. Preferred examples of the hydrophobic constituent unit may include stearyl methacrylate and lauryl methacrylate.

### [Other Constituent Units]

Constituent units except the constituent units represented by the formulae (1a) to (1c) may be introduced into the copolymer (P) to be used in the soft contact lens solution of the present disclosure to such an extent that the effects of the solution are not impaired. When other polymerizable monomers (other constituent units) described below are blended with the monomer composition to be used for synthesis of the copolymer (P), the blending ratio thereof may be appropriately selected to such an extent that the effects of the solution are not influenced. For example, when the nₐ represented in the formula (1a) forming the copolymer (P) is set to 100, the blending ratio is preferably 50 or less in terms of molar ratio.

Examples of the other polymerizable monomers that may be used for synthesis of the copolymer (P) may include a linear or branched alkyl (meth)acrylate, a cyclic alkyl (meth)acrylate, an aromatic group-containing (meth)acrylate, a styrene-based monomer, a vinyl ether monomer, a vinyl ester monomer, a hydrophilic hydroxy group-containing (meth)acrylate, an acid group-containing monomer, a nitrogen-containing group-containing monomer, an amino group-containing monomer, and a cationic group-containing monomer.

Examples of the linear or branched alkyl (meth)acrylate include methyl (meth)acrylate, ethyl (meth)acrylate, butyl (meth)acrylate, and 2-ethylhexyl (meth)acrylate.

An example of the cyclic alkyl (meth)acrylate is cyclohexyl (meth)acrylate.

Examples of the aromatic group-containing (meth)acrylate include benzyl (meth)acrylate and phenoxyethyl (meth)acrylate.

Examples of the styrene-based monomer include styrene, methylstyrene, and chloromethylstyrene.

Examples of the vinyl ether monomer include methyl vinyl ether, butyl vinyl ether, diethylene glycol monovinyl ether, and ethylene glycol monovinyl ether.

Examples of the vinyl ester monomer include vinyl acetate and vinyl propionate.

Examples of the hydrophilic hydroxy group-containing (meth)acrylate include polyethylene glycol (meth)acrylate, polypropylene glycol (meth)acrylate, 2-hydroxyethyl (meth)acrylate, 2-hydroxybutyl (meth)acrylate, 4-hydroxybutyl (meth)acrylate, and 2-hydroxypropyl (meth)acrylate.

Examples of the acid group-containing monomer include (meth)acrylic acid, styrenesulfonic acid, and (meth)acryloyloxyphosphonic acid.

Examples of the nitrogen-containing group-containing monomer include N-vinylpyrrolidone, N-vinylacetamide, and N-methyl-N-vinylacetamide.

Examples of the amino group-containing monomer include aminoethyl (meth)acrylate, dimethylaminoethyl (meth)acrylate, and N,N-dimethylaminopropyl (meth)acrylamide.

An example of the cationic group-containing monomer is 2-hydroxy-3-(meth)acryloyloxypropyltrimethylammonium chloride.

### [Molecular Weight of Copolymer (P)]

The copolymer (P) to be used in the soft contact lens solution of the present disclosure is a polymer having a weight-average molecular weight of from 5,000 to 2,000,000, preferably from 100,000 to 1,500,000. When the weight-average molecular weight is less than 5,000, the adsorptivity of the copolymer to the surface of a contact lens is not sufficient, and hence the intended effects (surface hydrophilicity, lubricity, and a lipid adhesion-suppressing ability) may not be expected. When the weight-average molecular weight is more than 2,000,000, it may be difficult to perform aseptic filtration required for producing a contact lens solution.

### [Synthesis Method for Copolymer (P)]

The copolymer (P) to be used in the soft contact lens solution of the present disclosure may be obtained by subjecting a blend of the above-mentioned monomers to radial polymerization. The synthesis of the copolymer (P) may be performed, for example, by subjecting a composition of the above-mentioned monomers to radical polymerization in the presence of a radical polymerization initiator under purging with an inert gas, such as nitrogen, carbon dioxide, argon, or helium, or under an atmosphere of the inert gas. The radical polymerization method may be performed by a known method, such as bulk polymerization, suspension polymerization, emulsion polymerization, or solution polymerization. The radial polymerization method is preferably solution polymerization from the viewpoint of, for example, purification. The copolymer (P) may be purified by a known purification method, such as a reprecipitation method, a dialysis method, or an ultrafiltration method.

Examples of the radical polymerization initiator may include an azo-based radical polymerization initiator, an organic peroxide, and a persulfate.

Examples of the azo-based radical polymerization initiator include 2,2-azobis(2-diaminopropyl) dihydrochloride, 2,2-azobis(2-(5-methyl-2-imidazolin-2-yl)propane) dihydrochloride, 4,4-azobis-(4-cyanovaleric acid), 2,2-azobisisobutylamide dihydrate, 2,2-azobis(2,4-dimethylvaleronitrile), and 2,2-azobisisobutyronitrile (AIBN).

Examples of the organic peroxide include t-butyl peroxyneodecanoate, benzoyl peroxide, diisopropyl peroxydicarbonate, t-butyl peroxy-2-ethylhexanoate, t-butyl peroxypivalate, t-butyl peroxydiisobutyrate, lauroyl peroxide, t-butyl peroxydecanoate, and succinic acid peroxide (=succinyl peroxide).

Examples of the persulfate include ammonium persulfate, potassium persulfate, and sodium persulfate.

Those radical polymerization initiators may be used alone or as a mixture thereof. A usage amount of the polymerization initiator is usually from 0.001 part by mass to 10 parts by mass, preferably from 0.01 part by mass to 5.0 parts by mass with respect to 100 parts by mass of the monomer composition.

The synthesis of the copolymer (P) may be performed in the presence of a solvent. Any solvent may be used as the solvent as long as the solvent dissolves the monomer composition and does not react therewith, and examples thereof may include water, an alcohol-based solvent, a ketone-based solvent, an ester-based solvent, a linear or cyclic ether-based solvent, and a nitrogen-containing solvent. Preferred examples of the solvent include water, an alcohol, and a mixed solvent thereof.

Examples of the alcohol-based solvent include methanol, ethanol, n-propanol, and isopropanol.

Examples of the ketone-based solvent include acetone, methyl ethyl ketone, and diethyl ketone.

An example of the ester-based solvent is ethyl acetate.

Examples of the linear or cyclic ether-based solvent include ethyl cellosolve and tetrahydrofuran.

Examples of the nitrogen-containing solvent include acetonitrile, nitromethane, and N-methyl pyrrolidone.

### <Copolymer Q>

The copolymer (Q) to be used in the soft contact lens solution of the present disclosure is a polyoxyethylene-polyoxypropylene block copolymer, has a number of moles added of ethylene oxide (EO) of from 120 to 200 and a number of moles added of propylene oxide (PO) of from 10 to 70, and has a content of the EO of from 60 to 90.

Examples of the polyoxyethylene-polyoxypropylene block copolymer include a polyoxyethylene-polyoxypropylene-polyoxyethylene triblock copolymer and a polyoxypropylene-polyoxyethylene-polyoxypropylene triblock copolymer. Of those, a polyoxyethylene-polyoxypropylene-polyoxyethylene triblock copolymer is preferred.

The number of moles added of the ethylene oxide (EO) in the copolymer (Q) falls within a range of preferably from 120 to 200, more preferably from 140 to 180. In addition, the number of moles added of the propylene oxide (PO) in the copolymer (Q) falls within a range of preferably from 10 to 70, more preferably from 20 to 40. In addition, the ranges (e.g., preferred ranges, more preferred ranges) of the number of moles added of the ethylene oxide (EO) and the number of moles added of the propylene oxide (PO) may be combined or changed. For example, the preferred range of the number of moles added of the ethylene oxide (EO) and the more preferred range of the number of moles added of the propylene oxide (PO) may be selected. When those numbers of moles added are too small, the copolymer (Q) may become so small as to enter into the contact lens to cause deformation. When those numbers of moles added are too large, the viscosity of the contact lens solution may be increased to make it difficult to perform aseptic filtration.

In addition, the content of the EO in the copolymer (Q) is preferably from 60 wt% to 90 wt%, more preferably from 70 wt% to 80 wt%. When the content of the EO is less than 60 wt%, it becomes difficult to impart sufficient effects (in particular, surface hydrophilicity and lubricity) to a contact lens. When the content of the EO is more than 90 wt%, the adsorptivity to a contact lens may be decreased.

### <Buffering Agent>

A buffering agent that may be used in a general contact lens solution and the like may be used as the buffering agent to be used in the soft contact lens solution of the present disclosure. Specific examples thereof may include hydrochloric acid, acetic acid, citric acid, sodium hydroxide, boric acid, a borate (e.g., borax), a phosphate (e.g., monosodium phosphate, disodium phosphate, monopotassium phosphate, or dipotassium phosphate), a citrate (e.g., sodium citrate), tris(hydroxymethyl)aminomethane, and hydrates thereof, and two or more kinds of these buffering agents are preferably used in combination. A phosphate buffer to be used in the soft contact lens solution of the present disclosure is not particularly limited as long as the phosphate buffer is a buffering agent that is dissolved in the soft contact lens solution so that phosphoric acid (phosphoric acid ions) is incorporated into the solution, and is preferably a buffer containing monosodium phosphate and disodium phosphate, or hydrates thereof.

The concentration of the buffering agent is from 0.01 w/v% to 1.5 w/v%, and more preferably falls within a range of from 0.05 w/v% to 1.0 w/v%. It is not preferred that the concentration be less than 0.01 w/v% because the buffering capacity of the buffering agent is low and it becomes difficult to control the pH of the contact lens solution. It is not preferred that the concentration be more than 1.5 w/v% because the solubility of other components is impaired. In addition, from the viewpoints of reducing irritation during wearing of the contact lenses and improving wearing comfort, it is preferred that the buffering agent be blended at any ratio so that the pH of the contact lens solution becomes from 3 to 9, more preferably from 4 to 8.

The pH of the contact lens solution as used herein means a value measured in accordance with The Japanese Pharmacopoeia Seventeenth Edition, General Tests 2.54, pH Determination.

### <Composition of Soft Contact Lens Solution of the Present Disclosure>

The soft contact lens solution of the present disclosure may be obtained by dissolving 0.001 w/v% to 1.0 w/v% of the copolymer (P), 0.002 w/v% to 2.0 w/v% of the copolymer (Q), and the buffering agent in water or a mixed solution of an alcohol, such as ethanol, n-propanol, or isopropanol, and water.

When the copolymer (P), the copolymer (Q), and the buffering agent are incorporated into the soft contact lens solution, lubricity, hydrophilicity, and a lipid adhesion-suppressing ability can be imparted to a contact lens, and hence satisfactory wearing comfort can be given to a contact lens wearer.

In addition to the copolymer (P) and the copolymer (Q), a tonicity agent, vitamins, amino acids, saccharides, a thickening agent, a refreshing agent, inorganic salts, an antioxidant, a stabilizer, a preservative, and the like, which may be used in a general contact lens solution and the like, may be blended as required in the soft contact lens solution of the present disclosure.

The blending amount of those components except the copolymer (P) and the copolymer (Q) is not particularly limited, and is preferably from 0.01 w/v% to 5.0 w/v%, more preferably from 0.05 w/v% to 4.0 w/v%, still more preferably from 0.1 w/v% to 3.0 w/v%.

An example of the tonicity agent is sodium chloride.

Examples of the vitamins include flavin adenine dinucleotide sodium, cyanocobalamin, retinol acetate, retinol palmitate, pyridoxine hydrochloride, panthenol, sodium pantothenate, and calcium pantothenate.

Examples of the amino acids include aspartic acid or salts thereof, and aminoethylsulfonic acid.

Examples of the saccharides include glucose, mannitol, sorbitol, xylitol, and trehalose.

Examples of the thickening agent include hydroxypropyl methylcellulose and hydroxyethyl cellulose.

Examples of the refreshing agent include menthol and camphor.

Examples of the inorganic salts include sodium chloride, potassium chloride, and sodium dihydrogen phosphate anhydride.

Examples of the antioxidant include tocopherol acetate, sodium hydrogen sulfite, sodium sulfite, ascorbic acid, and dibutyl hydroxytoluene.

Examples of the stabilizer include sodium edetate and glycine.

Examples of the preservative include benzalkonium chloride, chlorhexidine gluconate, potassium sorbate, and polyhexanide hydrochloride.

### <Product Form of Soft Contact Lens Solution of the Present Disclosure>

The following forms may be given as specific examples of product forms of the soft contact lens solution of the present disclosure. The specific examples include a contact lens shipping solution, a contact lens care product, a contact lens storage solution, a contact lens cleaning solution, and a contact lens cleaning storage solution. As used herein, the contact lens shipping solution means a solution in which a contact lens is immersed when a contact lens product is shipped. In addition, as used herein, the contact lens care product is not particularly limited as long as the care product is a solution to be used for the care of a soft contact lens product, and means, for example, a disinfecting solution, a cleaning solution, a storage solution, a cleaning storage solution, a disinfecting cleaning storage solution, or a wetting solution for a soft contact lens.

### (Configuration Example of Contact Lens Solution of the Present Disclosure)

Combinations of the respective constituent units of the copolymer (P) included in the contact lens solution of the present disclosure are exemplified below, but are not limited thereto.

Constituent unit represented by the formula (1a): constituent unit represented by the formula (1b): constituent unit represented by the formula (1c)
2-(Methacryloyloxy)ethyl 2-(trimethylammonio)ethyl phosphate: N,N-dimethylacrylamide: stearyl methacrylate
2-(Methacryloyloxy)ethyl 2-(trimethylammonio)ethyl phosphate: N,N-dimethylacrylamide: lauryl methacrylate

In addition, each of the constituent unit represented by the formula (1a), the constituent unit represented by the formula (1b), and the constituent unit represented by the formula (1c) in each of the above-mentioned combinations may be changed to the constituent unit in another combination to form the copolymer (P) included in the contact lens solution of the present disclosure.

### (Surface Treatment Method for Soft Contact Lens of the Present Disclosure)

One embodiment of the present disclosure is also directed to the above-mentioned surface treatment method for a soft contact lens including the step of bringing the soft contact lens solution of the present invention into contact with the surface of a soft contact lens. Examples of the contact may include immersing a soft contact lens in the soft contact lens solution of the present disclosure and spraying the soft contact lens solution of the present disclosure to the surface of a soft contact lens, but the contact is not particularly limited thereto.

### (Use of Copolymer (P), Copolymer (Q), and Buffering Agent of the Present Disclosure for Production of Soft Contact Lens Solution)

One embodiment of the present disclosure is also directed to the above-mentioned use of the copolymer (P), the copolymer (Q), and the buffering agent for production of the soft contact lens solution of the present disclosure.

The soft contact lens solution of the present disclosure is described below in more detail by way of Examples and Comparative Examples, but the soft contact lens solution of the present disclosure is not limited thereto.

### <Copolymer (P)>

The following copolymer (P1) to copolymer (P4) were each used as the copolymer (P).

In Examples, the weight-average molecular weight of the copolymer (P) was determined by dissolving 5 mg of each of the resultant copolymers in a methanol/chloroform mixed liquid (80:20) to provide a sample solution. The following analysis conditions were used.
Column: PLgel-mixed-C
Standard reference material: polyethylene glycol
Detector: differential refractometer RI-8020 (manufactured by Tosoh Corporation)
Calculation method for weight-average molecular weight: molecular weight calculation program (GPC program for SC-8020)
Flow rate: 1 mL per minute
Injection amount: 100 µL
Column oven: constant temperature around 40°C

### [Copolymer (P1)]

A copolymer using, as constituent units, 2-(methacryloyloxy)ethyl 2-(trimethylammonio)ethyl phosphate represented by the general formula (1a") serving as a PC monomer (PC constituent unit), N,N-dimethylacrylamide serving as an amide monomer (amide constituent unit), and stearyl methacrylate serving as a hydrophobic monomer (hydrophobic constituent unit)
Ratio of numbers of constituent units: [(1a)/(1b)/(1c)]=100/90/10
Weight-average molecular weight: 1,000,000 [Copolymer (P2)]

A copolymer using, as constituent units, 2-(methacryloyloxy)ethyl 2-(trimethylammonio)ethyl phosphate represented by the general formula (1a") serving as a PC monomer, N,N-dimethylacrylamide serving as an amide monomer, and stearyl methacrylate serving as a hydrophobic monomer
Ratio of numbers of constituent units: [(1a)/(1b)/(1c)]|=100/223/10
Weight-average molecular weight: 1,200,000 [Copolymer (P3)]

A copolymer using, as constituent units, 2-(methacryloyloxy)ethyl 2-(trimethylammonio)ethyl phosphate represented by the general formula (1a") serving as a PC monomer, N,N-dimethylacrylamide serving as an amide monomer, and stearyl methacrylate serving as a hydrophobic monomer
Ratio of numbers of constituent units: [(1a)/(1b)/(1c)]|=100/34/9
Weight-average molecular weight: 700,000 [Copolymer (P4)]

A copolymer using, as constituent units, 2-(methacryloyloxy)ethyl 2-(trimethylammonio)ethyl phosphate represented by the general formula (1a") serving as a PC monomer, N,N-dimethylacrylamide serving as an amide monomer, and lauryl methacrylate serving as a hydrophobic monomer
Ratio of numbers of constituent units: [(1a)/(1b)/(1c)]|=100/90/20
Weight-average molecular weight: 1,000,000

### <Polymer for Comparison to Copolymer (P)>

The following polymers (A) to (C) were each used as a polymer for comparison to the copolymer (P).

### [Polymer (A)]

2-Methacryloyloxyethyl phosphorylcholine homopolymer (weight-average molecular weight: 200,000) obtained by the method described in Examples of JP 08-333421 A

### [Polymer (B)]

Commercially available N,N-dimethylacrylamide homopolymer (Poly(N,N-dimethylacrylamide), DDMAT Terminated (product name) manufactured by Sigma-Aldrich Japan, number-average molecular weight: 10,000)

### [Polymer (C)]

Commercially available lauryl methacrylate homopolymer (Lauryl polymethacrylate (product name) manufactured by Sigma-Aldrich Japan, weight-average molecular weight: 470,000)

### <Copolymer (Q)>

The following copolymers (Q1) to (Q3) were used as the copolymer (Q).

### [Copolymer (Q1)]

Commercially available polyoxyethylene-polyoxypropylene-polyoxyethylene block copolymer (PLONON (Registered trademark) #188P (product name) manufactured by NOF CORPORATION, number of moles added of EO: 160, number of moles added of PO: 30, content of EO: 80 wt%)

### [Copolymer (Q2)]

Commercially available polyoxyethylene-polyoxypropylene-polyoxyethylene block copolymer (PLONON (Registered trademark) #208 (product name) manufactured by NOF CORPORATION, number of moles added of EO: 150, number of moles added of PO: 35, content of EO: 80 wt%)

### [Copolymer (Q3)]

Commercially available polyoxyethylene-polyoxypropylene-polyoxyethylene block copolymer (UNILUBE (Registered trademark) 70DP-950B (product name) manufactured by NOF CORPORATION, number of moles added of EO: 200, number of moles added of PO: 70, content of EO: 70 wt%)

### <Polymer for Comparison to Copolymer (Q)>

The following polymers (D) and (E) were each used as a polymer for comparison to the copolymer (Q).

### [Polymer (D)]

Commercially available ethylene glycol homopolymer (Polyethylene glycol 10000 (product name) manufactured by Sigma-Aldrich Japan, weight-average molecular weight: 9,000)

### [Polymer (E)]

Commercially available propylene glycol homopolymer (Polypropylene glycol 4,000 (product name) manufactured by Alfa Aesar), weight-average molecular weight: 4,000)

### <Preparation of Contact Lens Solution>

### [Example 1]

About 80 g of purified water was weighed, and 0.01 g of the copolymer (P1), 0.02 g of the copolymer (Q1), and 0.83 g of sodium chloride, 0.599 g of sodium hydrogen phosphate dodecahydrate, and 0.053 g of sodium dihydrogen phosphate dihydrate each serving as a buffering agent were dissolved in the purified water to provide a total amount of 100 mL. The resultant was filtered to provide a sterile contact lens solution. The appearance and properties of the contact lens solution of Example 1 are shown in Table 1.

### [Examples 2 to 15]

Sterile contact lens solutions were prepared in accordance with the same procedure as that in Example 1 except that components of the kinds and amounts shown in Tables 1 and 2 were used. The appearance and properties of the contact lens solution of each of Examples 2 to 15 are shown in Tables 1 and 2.

### [Comparative Examples 1 to 5]

Sterile contact lens solutions were prepared in accordance with the same procedure as that in Example 1 except that components of the kinds and amounts shown in Table 3 were used. The appearance and properties of the contact lens solution of each of Comparative Examples 1 to 5 are shown in Table 3.

**Table 1**

| | | Example 1 | Example 2 | Example 3 | Example 4 | Example 5 | Example 6 | Example 7 |
|---|---|---|---|---|---|---|---|---|
| Copolymer (P) | Copolymer (P1) | 0.01 g | 0.01 g | 0.01 g | 1.0 g | 0.1 g | 0.001 g | 0.01 g |
| | Copolymer (P2) | | | | | | | |
| | Copolymer (P3) | | | | | | | |
| | Copolymer (P4) | | | | | | | |
| Polymer for comparison to copolymer (P) | Polymer (A) | | | | | | | |
| | Polymer (B) | | | | | | | |
| | Polymer (C) | | | | | | | |
| Copolymer (Q) | Copolymer (Q1) | 0.02 g | | | 0.02 g | 0.02 g | 0.02 g | 2.0 g |
| | Copolymer (Q2) | | 0.02 g | | | | | |
| | Copolymer (Q3) | | | 0.02 g | | | | |
| Polymer for comparison to copolymer (Q) | Polymer (D) | | | | | | | |
| | Polymer (E) | | | | | | | |
| Buffering agent | Sodium hydrogen phosphate dodecahydrate | 0.599 g | 0.599 g | 0.599 g | 0.599 g | 0.599 g | 0.599 g | 0.599 g |
| | Sodium dihydrogen phosphate dihydrate | 0.053 g | 0.053 g | 0.053 g | 0.053 g | 0.053 g | 0.053 g | 0.053 g |
| | Boric acid | | | | | | | |
| | Borax | | | | | | | |
| Others | Sodium chloride | 0.83 g | 0.83 g | 0.83 g | 0.83 g | 0.83 g | 0.83 g | 0.83 g |
| | Purified water | Total amount is set to 100 mL | | | | | | |
| Analysis results | Properties·appearance | Colorless and transparent | Colorless and transparent | Colorless and transparent | Colorless and transparent | Colorless and transparent | Colorless and transparent | Colorless and transparent |
| Evaluation results | Surface lubricity (assuming start of wearing) | 7 | 8 | 6 | 10 | 9 | 6 | 6 |
| | Surface lubricity (assuming during wearing) | 7 | 6 | 6 | 10 | 8 | 6 | 6 |
| | Surface hydrophilicity (assuming start of wearing) | 23 | 24 | 17 | 31 | 29 | 17 | 25 |
| | Surface hydrophilicity (assuming during wearing) | 14 | 13 | 11 | 23 | 19 | 11 | 12 |
| | Lipid adhesion-suppressing rate (%) | 14 | 15 | 22 | 20 | 16 | 14 | 51 |

**Table 2**

| | | Example 8 | Example 9 | Example 10 | Example 11 | Example 12 | Example 13 | Example 14 | Example 15 |
|---|---|---|---|---|---|---|---|---|---|
| Copolymer (P) | Copolymer (P1) | 0.01 g | 0.01 g | | | | 0.01 g | 0.01 g | |
| | Copolymer (P2) | | | 0.01 g | | | | | 0.01 g |
| | Copolymer (P3) | | | | 0.01 g | | | | |
| | Copolymer (P4) | | | | | 0.01 g | | | |
| Polymer for comparison to copolymer (P) | Polymer (A) | | | | | | | | |
| | Polymer (B) | | | | | | | | |
| | Polymer (C) | | | | | | | | |
| Copolymer (Q) | Copolymer (Q1) | 0.2 g | 0.002 g | 0.02 g | 0.02 g | 0.02 g | 0.02 g | | 0.02 g |
| | Copolymer (Q2) | | | | | | | 0.02 g | |
| | Copolymer (Q3) | | | | | | | | |
| Polymer for comparison to copolymer (Q) | Polymer (D) | | | | | | | | |
| | Polymer (E) | | | | | | | | |
| Buffering agent | Sodium hydrogen phosphate dodecahydrate | 0.599 g | 0.599 g | 0.599 g | 0.599 g | 0.599 g | | | |
| | Sodium dihydrogen phosphate dihydrate | 0.053 g | 0.053 g | 0.053 g | 0.053 g | 0.053 g | | | |
| | Boric acid | | | | | | 0.4 g | 0.4 g | 0.4 g |
| | Borax | | | | | | 0.0198 g | 0.0198 g | 0.0198 g |
| Others | Sodium chloride | 0.83 g | 0.83 g | 0.83 g | 0.83 g | 0.83 g | 0.7 g | 0.7 g | 0.7 g |
| | Purified water | Total amount is set to 100 mL | | | | | | | |
| Analysis results | Properties·appearance | Colorless and transparen t | Colorless and transparen t | Colorless and transparen t | Colorless and transparen t | Colorless and transparen t | Colorless and transparen t | Colorless and transparen t | Colorless and transparen t |
| Evaluation results | Surface lubricity (assuming start of wearing) | 6 | 7 | 6 | 6 | 6 | 7 | 7 | 7 |
| | Surface lubricity (assuming during wearing) | 6 | 6 | 6 | 6 | 6 | 6 | 6 | 7 |
| | Surface hydrophilicity (assuming start of wearing) | 22 | 22 | 19 | 21 | 19 | 24 | 21 | 20 |
| | Surface hydrophilicity (assuming during wearing) | 11 | 10 | 11 | 12 | 11 | 14 | 13 | 11 |
| | Lipid adhesion-suppressing rate (%) | 37 | 11 | 20 | 19 | 12 | 14 | 13 | 14 |

**Table 3**

| | | Comparative Example 1 | Comparative Example 2 | Comparative Example 3 | Comparative Example 4 | Comparative Example 5 |
|---|---|---|---|---|---|---|
| Copolymer (P) | Copolymer (P1) | | | | | |
| | Copolymer (P2) | | | | | |
| | Copolymer (P3) | | | | | |
| | Copolymer (P4) | | | | | |
| Polymer for comparison to copolymer (P) | Polymer (A) | 0.01 g | 0.01 g | | | 0.1 g |
| | Polymer (B) | | | 0.01 g | | |
| | Polymer (C) | | | | 0.01 g | |
| Copolymer (Q) | Copolymer (Q1) | | | | | |
| | Copolymer (Q2) | | | | | |
| | Copolymer (Q3) | | | | | |
| Polymer for comparison to copolymer (Q) | Polymer (D) | 0.02 g | | 0.02 g | | 0.2 g |
| | Polymer (E) | | 0.02 g | | 0.02 g | |
| Buffering agent | Sodium hydrogen phosphate dodecahydrate | 0.599 g | 0.599 g | 0.599 g | 0.599 g | |
| | Sodium dihydrogen phosphate dihydrate | 0.053 g | 0.053 g | 0.053 g | 0.053 g | |
| | Boric acid | | | | | 0.4 g |
| | Borax | | | | | 0.0198 g |
| Others | Sodium chloride | 0.83 g | 0.83 g | 0.83 g | 0.83 g | 0.7 g |
| | Purified water | Total amount is set to 100 mL | | | | |
| Analysis results | Properties·appearance | Colorless and transparent | Insoluble | Colorless and transparent | Insoluble | Colorless and transparent |
| Evaluation results | Surface lubricity (assuming start of wearing) | 4 | - | 2 | - | 5 |
| | Surface lubricity (assuming during wearing) | 1 | - | 1 | - | 2 |
| | Surface hydrophilicity (assuming start of wearing) | 6 | - | 5 | - | 7 |
| | Surface hydrophilicity (assuming during wearing) | 2 | - | 2 | - | 1 |
| | Lipid adhesion-suppressing rate (%) | 5 | - | 2 | - | 7 |

Sodium chloride: manufactured by Otsuka Pharmaceutical Co., Ltd.
Sodium hydrogen phosphate dodecahydrate: manufactured by FUJIFILM Wako Pure Chemical Corporation
Sodium dihydrogen phosphate dihydrate: manufactured by FUJIFILM Wako Pure Chemical Corporation
Boric acid: manufactured by FUJIFILM Wako Pure Chemical Corporation
Borax: manufactured by FUJIFILM Wako Pure Chemical Corporation
Purified water: manufactured by KENEI Pharmaceutical Co., Ltd.

### <Recognition of Improving Effect on Lubricity of Contact Lens by Contact Lens Solution>

The evaluation of the improvement in lubricity of a contact lens by the contact lens solution of each of Examples and Comparative Examples was performed in accordance with the following procedure. Menicon 1Day (Registered trademark) (manufactured by Menicon Co., Ltd.) was used for evaluating the improvement in lubricity of a contact lens.

### (Procedure)

1) The contact lens solution of Example 1 was prepared.
2) One contact lens was taken out from a blister pack and placed in a 15 mL centrifuge tube.
3) 10 mL of ISO physiological saline was added to the centrifuge tube of the step 2), and the tube was shaken overnight.
4) After the shaking in the step 3), the ISO physiological saline was removed, and 10 mL of the contact lens solution prepared in the step 1) was added to the resultant.
5) Autoclave treatment was performed under the conditions of 121°C for 20 minutes.
6) After the resultant was cooled to room temperature, the contact lens was taken out, placed on an index finger, and subjected to evaluation of lubricity assuming start of wearing of the contact lens.
7) After the step 6), the contact lens was temporarily immersed in the solution prepared in the step 1), a water film on the surface of the contact lens was wiped off, and the contact lens was placed on an index finger and subjected to evaluation of lubricity assuming during wearing of the contact lens.

### (Composition of ISO Physiological Saline)

| | |
|---|---|
| Sodium chloride | 8.3 g |
| Sodium hydrogen phosphate dodecahydrate | 0.528 g |
| Sodium dihydrogen phosphate dihydrate | 0.528 g |
| Ion-exchanged water | Total amount is set to 100 mL |

### (ISO 18369-3:2006, Ophthalmic Optics-Contact Lenses Part 3: Measurement Methods)

Lubricity was evaluated on a scale of 1 to 10 points. The evaluation point in the case of the evaluation of "most excellent in lubricity" was defined as 10, and the evaluation point in the case of the evaluation of "there is no lubricity" was defined as 1. The value of 6.0 or more was evaluated as "excellent in surface lubricity," and the value of 8.5 or more was evaluated as "particularly excellent in surface lubricity."

The contact lens solutions of Examples 2 to 15 and Comparative Examples 1 to 5 were also evaluated in accordance with the above-mentioned procedure.

### <Recognition of Improving Effect on Surface Hydrophilicity of Contact Lens by Contact Lens Solution>

The evaluation of the improvement in surface hydrophilicity of a contact lens by the contact lens solution of each of Examples and Comparative Examples was performed in accordance with the following procedure. Menicon 1Day (Registered trademark) (manufactured by Menicon Co., Ltd.) was used for evaluating the improving effect on surface hydrophilicity of a contact lens.

### (Procedure)

1) The same procedure as that in each of the steps 1) to 5) described in the <Recognition of Improving Effect on Lubricity of Contact Lens> section was performed.
2) A contact lens was taken out, and the time until the water film on the surface of the lens was broken (tear film breakup time (BUT)) was measured with a stopwatch. The measurement result was used as the evaluation of surface hydrophilicity assuming start of wearing of the contact lens.
3) Another contact lens was immersed in 2 mL of ISO physiological saline, and the tube was shaken for 6 hours.
4) After 6 hours, the contact lens was taken out, and the BUT was measured with a stopwatch. The measurement result was used as the evaluation of surface hydrophilicity assuming during wearing of the contact lens.

The BUT of 10 seconds or more was evaluated as "excellent in surface hydrophilicity," and the BUT of 15 seconds or more was evaluated as "particularly excellent in surface hydrophilicity."

The contact lens solutions of Examples 2 to 15 and Comparative Examples 1 to 5 were also evaluated in accordance with the above-mentioned procedure.

### <Recognition of Improving Effect on Lipid Adhesion-suppressing Ability of Contact Lens by Contact Lens Solution>

The evaluation of the lipid adhesion-suppressing ability of a contact lens by the contact lens solution of each of Examples and Comparative Examples was performed in accordance with the following procedure. CLARITI 1Day (Registered trademark) (manufactured by CooperVision Japan) was used for investigating the improving effect on the lipid adhesion-suppressing ability of a contact lens.

### (Procedure)

### Preparation of Artificial Eye Lipid

1) 0.5 g of mixed lipid having the following composition was mixed into 100 mL of a phosphate-borate buffer described below.
2) The mixture was suspended at 60°C with a homomixer.
3) The resultant was adjusted to a pH of 7.0 with 1 N hydrochloric acid.

### (Composition of Mixed Lipid)

| | |
|---|---|
| Oleic acid | 0.06 g |
| Linolenic acid | 0.06 g |
| Palmitic acid | 0.06 g |
| Tripalmitic acid | 0.81 g |
| Cetyl alcohol | 0.20 g |
| Cetyl myristate | 0.81 g |
| Cholesterol | 0.08 g |
| Cholesterol palmitate | 0.08 g |
| Lecithin (derived from egg) | 2.83 g |

### (Composition of Phosphate-Borate Buffer)

| | |
|---|---|
| Sodium chloride | 2.25 g |
| Potassium dihydrogen phosphate | 1.25 g |
| Sodium tetraborate decahydrate | 5.65 g |
| Ion-exchanged water | Total amount is set to 250 mL |

Reference: Kaoru Iwai; Mari Moriyama; Masaki Imayasu; Hidenari Tanaka: Lipid Adsorption to Contact Lens Materials., Journal of Japan Contact Lens Society, 1995, 37(1), 58-61.

### (Evaluation)

1) The same procedure as that in each of the steps 1) to 5) described in the <Recognition of Improving Effect on Surface Lubricity of Contact Lens> section was performed.
2) After the contact lens was cooled to room temperature, the contact lens was immersed in 4 mL of artificial eye lipid per contact lens for 4 hours.
3) The contact lens was taken out and lightly rinsed with the ISO physiological saline described above, and moisture was removed.
4) The contact lens was immersed in 3 mL of an extract liquid (ethanol/diethyl ether=1/1 (v/v)).
5) Ultrasonic treatment was performed for 10 minutes.
6) 2 mL of the extract liquid was taken into a test tube, and the solvent was evaporated at 90°C.
7) 0.4 g of concentrated sulfuric acid was added to the test tube of the step 6), and the mixture was heated at 90°C for 15 minutes.
8) After the test tube of the step 7) was cooled to room temperature, 2.5 mL of the following vanillin solution was added to the test tube.
9) After the resultant was held at 37°C for 10 minutes, an absorbance at 540 nm was measured.

### (Vanillin solution)

| | |
|---|---|
| Vanillin | 0.12 g |
| Ion-exchanged water | 20 mL |
| Phosphoric acid | 80 mL |

The resultant absorbance was checked against a calibration curve to calculate the lipid adhesion amount of lipid to the contact lens. The lipid adhesion-suppressing rate was calculated based on the lipid adhesion amount performed in the same manner through use of ISO physiological saline as a control solution of the contact lens solution. The case of the lipid adhesion-suppressing rate of 10% or more was evaluated as "there is an adhesion-suppressing ability," and the case of the lipid adhesion-suppressing rate of more than 15% was evaluated as "excellent in adhesion-suppressing ability."

The contact lens solutions of Examples 2 to 15 and Comparative Examples 1 to 5 were also evaluated in accordance with the above-mentioned procedure.

### <Evaluation Results>

### (Improving Effect on Surface Lubricity of Contact Lens)

The surface lubricity of the contact lens treated with the contact lens solution of each of Examples 1 to 15 resulted in being excellent or most excellent even at the start of wearing and during wearing. In particular, the surface lubricity of the contact lens treated with the contact lens solution of Example 4 resulted in being most excellent in lubricity even at the start of wearing and during wearing. Further, it was recognized that the contact lens solution did not impart deformability to a contact lens.

Meanwhile, the improvement in surface lubricity of the contact lens treated with the contact lens solution of each of Comparative Examples 1 to 5 could not be recognized.

Thus, the contact lens solution of each of Examples 1 to 15 can impart (durable) excellent surface lubricity to a contact lens even during wearing.

### (Improving Effect on Surface Hydrophilicity of Contact Lens)

The surface hydrophilicity of the contact lens treated with the contact lens solution of each of Examples 1 to 15 resulted in being excellent or most excellent even at the start of wearing and during wearing.

Meanwhile, the improvement in surface hydrophilicity of the contact lens treated with the contact lens solution of each of Comparative Examples 1 to 5 could not be recognized.

Thus, the contact lens solution of each of Examples 1 to 15 can impart (durable) excellent surface hydrophilicity to a contact lens even during wearing.

### (Improving Effect on Lipid Adhesion-suppressing Ability of Contact Lens)

The lipid adhesion-suppressing ability of the contact lens treated with the contact lens solution of each of Example 1 to 15 resulted in being "excellent" or "present".

Meanwhile, the lipid adhesion-suppressing ability of the contact lens treated with the contact lens solution of each of Comparative Examples 1 to 5 could not be recognized.

Thus, the contact lens solution of each of Examples 1 to 15 can impart a lipid adhesion-suppressing ability to a contact lens.

As described above, the contact lens solution of the present disclosure can impart durable surface hydrophilicity, durable lubricity, and a lipid adhesion-suppressing ability to a contact lens through simple treatment (e.g., immersion of the contact lens in the solution), and hence can impart satisfactory wearing comfort to a contact lens wearer during wearing. Further, this satisfactory wearing comfort can be continued for a long period of time.

### Industrial Applicability

The contact lens solution that imparts surface hydrophilicity, lubricity, and a lipid adhesion-suppressing ability to a contact lens can be provided.

## Claims

1. A soft contact lens solution, comprising:
0.001 w/v% to 1.0 w/v% of a copolymer (P) containing constituent units represented by the following formulae (1a) to (1c), having a ratio "na:nb:nc" of numbers of the constituent units of 100:10 to 400:2 to 50, and having a weight-average molecular weight of from 5,000 to 2,000,000;
0.002 w/v% to 2.0 w/v% of a copolymer (Q) being a polyoxyethylene-polyoxypropylene block copolymer, having a number of moles added of ethylene oxide (EO) of from 120 to 200 and a number of moles added of propylene oxide (PO) of from 10 to 70, and having a content of the EO of from 60 wt% to 90 wt%; and
a buffering agent: 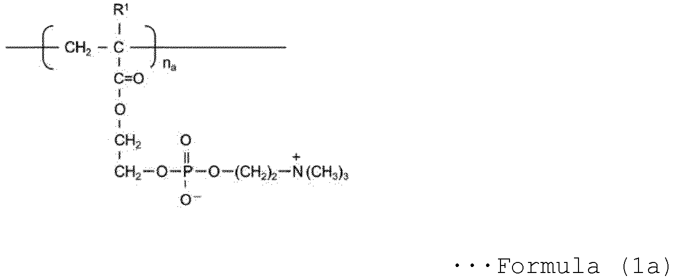 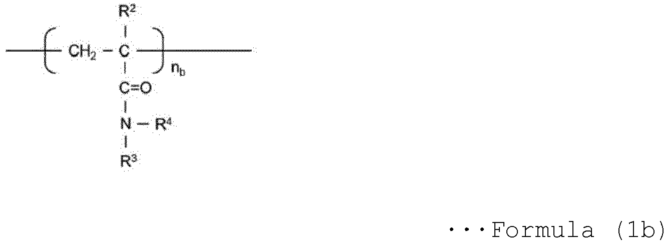 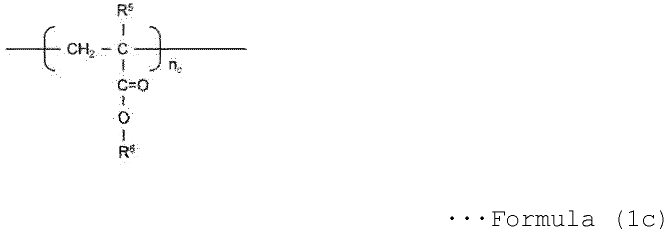 where R¹, R², and R⁵ each independently represent a hydrogen atom or a methyl group, R³ and R⁴ each independently represent a hydrogen atom, a methyl group, or an ethyl group, or are bonded to each other to represent a morpholino group, and R⁶ represents a monovalent hydrocarbon group having 12 to 24 carbon atoms.

2. The soft contact lens solution according to claim 1, wherein the buffering agent is a phosphate buffer.
